# EUROPÄISCHE PATENTSCHRIFT

(11) **EP 3 291 769 B1**
(45) Veröffentlichungstag und Bekanntmachung des Hinweises auf die Patenterteilung: **14.04.2021**
(21) Anmeldenummer: 16729040.2
(22) Anmeldetag: 03.05.2016
(51) Int. Cl.: A61F 2/44, A61F 2/46

(54) **ZWISCHENWIRBEL-IMPLANTAT**
INTERBODY IMPLANT
IMPLANT INTERVERTÉBRAL

(30) Priorität: 05.05.2015 CH 6182015
(43) Veröffentlichungstag der Anmeldung: 14.03.2018
(73) Patentinhaber: RS-Technik Cad-Cam GmbH, 78253 Eigeltingen-Honstetten (DE)
(72) Erfinder: SCHAFHÄUTLE, Roland, 78253 Eigeltingen-Honstetten (DE)
(74) Vertreter: Römpler, Aldo
(86) Internationale Anmeldenummer: PCT/IB2016/000586
(87) Internationale Veröffentlichungsnummer: WO 2016/178078

(56) Entgegenhaltungen:
- WO-A1-2013/025876
- WO-A1-2013/148176
- US-A1- 2010 292 796
- US-A1- 2011 282 453
- US-A1- 2014 257 484
- US-B1- 8 715 351

## Beschreibung

Die vorliegende Erfindung betrifft ein Zwischenwirbel-Implantat mit an benachbarten Wirbelkörpern anlegbaren Stützflächen, die bezüglich ihrer Lage zueinander verstellbar sind. Weiter bezieht sie sich auf ein Einbringinstrument zur Implantierung dieses Zwischenwirbel-Implantats. Insbesondere geht es um ein Zwischenwirbel-Implantat mit einer Spreizvorrichtung zum Auseinanderbewegen der an benachbarten Wirbelkörpern der menschlichen Wirbelsäule anlegbaren Stützflächen. In der englischen Sprache werden derartige Zwischenwirbel-Implantate auch als Cage bezeichnet. Sie werden dort eingesetzt, wo Zwischenwirbelkörper entfernt werden müssen. Mittels eines Zwischenwirbel-Implantats kann verhindert werden, dass sich der Zwischenwirbelraum schliesst und die Wirbelkörper unkontrolliert aufeinanderliegen. Letzteres kann zu Fehlstellungen der Wirbelsäule und in der Folge zu weiteren Schädigungen derselben führen, die dem Patienten erneute Beschwerden und Schmerzen bereiten können.

Operativ ergeben sich dabei folgende Probleme: erstens die Wahl eines Zwischenwirbel-Implantates passender Grösse, vornehmlich bezüglich der Dicke, und zweitens dessen Einführung und Positionierung. Ein zu kleines Zwischenwirbel-Implantat lässt sich zwar leichter einführen, erfüllt aber seinen Zweck nur in ungenügender Weise.

Aus diesem Grund wurden spreizbare Zwischenwirbel-Implantate geschaffen. Diese können in einer Grundstellung kleineren Querschnitts eingeführt und erst im Zwischenwirbelraum zu einer Stützstellung grösseren Querschnitts geweitet werden. Hierzu gibt es verschiedene Konstruktionen. Ein solches Zwischenwirbel-Implantat war früher im Längsschnitt annähernd U-förmig. Das heisst, es wies zwei längere U-Stege auf, die jeweils eine Stützfläche bilden, welche durch einen kürzeren U-Steg miteinander verbunden waren, der als Verbindungssteg diente. Die beiden längeren U-Stege konnten keilförmig auseinander getrieben werden, wodurch sich der Querschnitt des Zwischenwirbel-Implantats weitete. Durch dieses keilförmige Spreizen veränderte sich jedoch auch die Winkellage der beiden Stützflächen zueinander. Um dem vorzubeugen schlägt US 2014/0257484 ein Zwischenwirbel-Implantat mit zwei durch Keile auseinanderdrückbaren Spreizelementen vor. Diese sind mittels eines in eine U-förmige Aufnahme eingreifenden Bolzen geführt, wobei letzterer zu einem weiteren Bauteil gehört. Sobald die Spreizelemente in die Stützstellung auseinander gedrückt werden berühren sie einander nicht mehr. Deren Ausrichtung ist dadurch ungenügend sichergestellt. Der nächstliegende Stand der Technik geht aus WO 2013/148 176 hervor. Auch diese Konstruktion vermag hinsichtlich der sicheren Positionierbarkeit und der allfälligen Rückstellbarkeit nicht zu überzeugen. Auf der Grundlage dieser Erkenntnisse setzt sich die Erfindung die Aufgabe, ein Zwischenwirbel-Implantat zu schaffen, das einerseits in einer Grundstellung kleineren Querschnitts in einen Zwischenwirbelraum der menschlichen Wirbelsäule einführbar ist, andererseits aber dieser Querschnitt zu einer Stützstellung bewegbar ist, bei der die beiden Stützflächen optimal ausgerichtet sind.

Das erfindungsgemässe Implantat entspricht den kennzeichnenden Merkmalen des Patentanspruchs 1. Das Einbringinstrument zur Implantierung dieses Zwischenwirbel-Implantats geht aus Patentanspruch 5 hervor. Weitere vorteilhafte Ausbildungen des Erfindungsgedankens sind aus den abhängigen Patentansprüchen ersichtlich.

Nachfolgend werden bevorzugte Ausführungsbeispiele der Erfindung anhand der Zeichnung näher beschrieben.
- Fig. 1: zeigt ein Ausführungsbeispiel des Zwischenwirbel-Implantats in einer geschlossenen Grundstellung;
- Fig. 2: zeigt dasselbe Zwischenwirbel-Implantat in einer Sprengdarstellung;
- Fig. 3: zeigt dasselbe Zwischenwirbel-Implantat in einer offenen Stützstellung;
- Fig. 4: zeigt Beispiele von Oberflächenstrukturen des Zwischenwirbel-Implantats;
- Fig. 5 - 8: zeigen Beispiele eines Instruments zur Einführung und Verstellung des erfindungsgemässen Zwischenwirbel-Implantats.

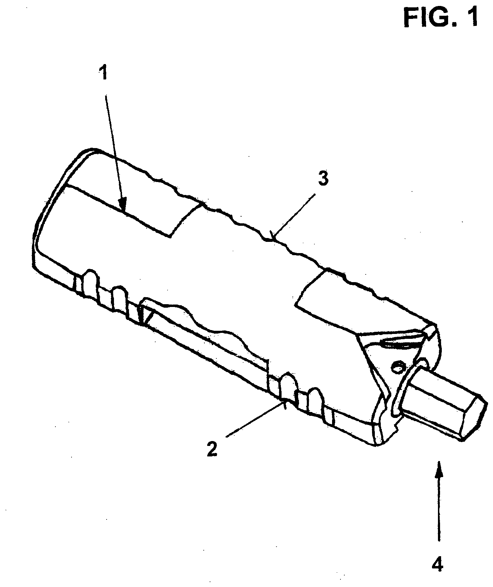

Das Ausführungsbeispiel des Zwischenwirbel-Implantats 1 ist nach Fig. 1 in der geschlossenen Grundstellung sehr kompakt und in sich geschlossen. Die beiden an benachbarten Wirbelkörpern der menschlichen Wirbelsäule anlegbaren Stützflächen 2 und 3 können, wie an sich bekannt, eine Oberflächenstruktur aufweisen. An einer Schmalseite des Zwischenwirbel-Implantats 1 ist ein Führungselement 4 zu sehen, auf den im Folgenden noch eingegangen wird.

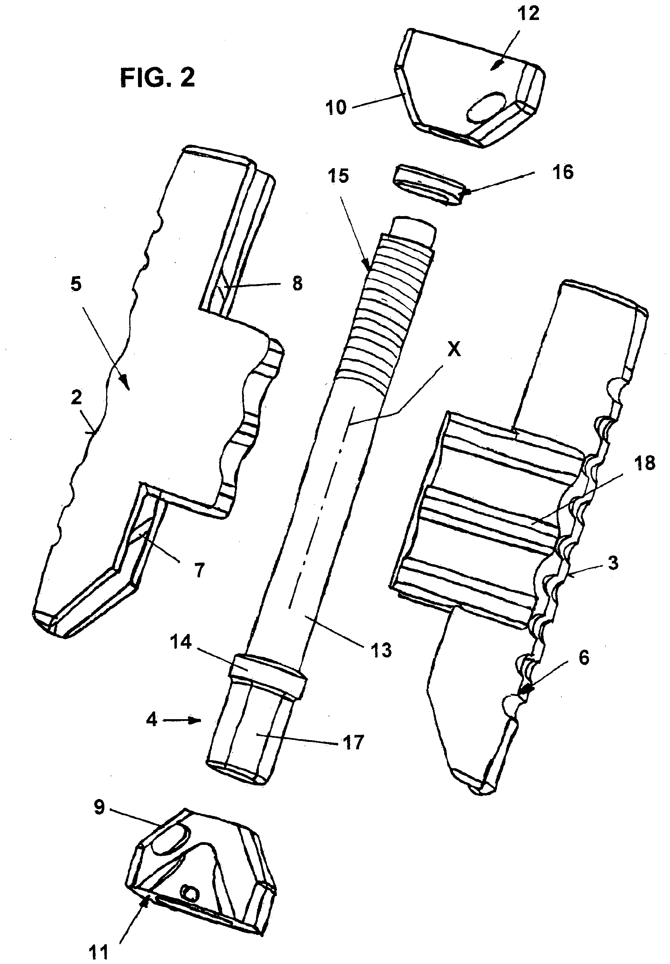

Anhand der Sprengdarstellung desselben Zwischenwirbel-Implantats 1 in Fig. 2 werden sowohl der Aufbau als auch die Funktion der verschiedenen Bauteile deutlich. Die erste Stützfläche 2 ist Teil eines ersten Spreizelementes 5 und die zweite Stützfläche 3 ist Teil eines zweiten Spreizelementes 6. Diese beiden Spreizelemente 5 und 6 sind schalenförmig. Mindestens eines dieser Spreizelemente, vorzugsweise jedoch beide, weisen an der Innenseite jeweils zwei Anschläge 7 und 8 auf. Bei dieser Ausführung sind diese als Schrägen ausgebildet und spitzwinklig zu den schmalen Enden der Spreizelemente 5 und 6 ausgerichtet, zum Beispiel in einem Winkel von 45°. Die Anschläge 7 und 8 sind dazu bestimmt, mit entsprechenden Keilflächen 9 und 10 von zwei Keilelementen 11 und 12 zusammenzuspielen. Diese sind ebenfalls spitzwinklig zu einer Achse X ausgerichtet, die der Längsachse eines Verstellelementes 13 entspricht. Die beiden Keilelemente 11 und 12 sind an diesem stabförmigen Verstellelement 13 angeordnet. Das erste Keilelement 11 ist in Richtung des Führungselementes 4 mittels eines Ringflansches 14 verschiebefest auf dem Verstellelement 13 gehalten. Das zweite Keilelement 12 weist ein Innengewinde auf und ist entlang einer Achse X verschiebbar auf einem Teil des Verstellelementes 13 angeordnet, das ein Aussengewinde 15 aufweist und dort mittels einer Endkappe 16 gehalten. Das Verstellelement 13 kann somit auch als Spindel bezeichnet werden. Das Führungselement 4, das einstückig am Verstellelement 13 ausgeformt sein kann, ist in diesem Beispiel als Bolzen mit dem Profil eines Mehrkants 17 ausgebildet, insbesondere eines Aussensechskants. Alternativ wäre auch eine Ausführung mit Innenmehrkant denkbar. Die beiden Spreizelemente 5 und 6 greifen unter anderem mittels Führungen 18 ineinander, die an den jeweils gleitend einander zugeordneten Flächen dieser beiden Spreizelemente 5 und 6 ausgeformt und rechtwinklig zur Achse X des Verstellelementes 13 ausgerichtet sind. Im vorliegenden Beispiel handelt es sich bei den Führungen 18 um Nuten und/oder Rillen. Denkbar wären beispielsweise aber auch Führungen in Form von hinterschnittenen Nuten und in diese eingreifenden Profilen.

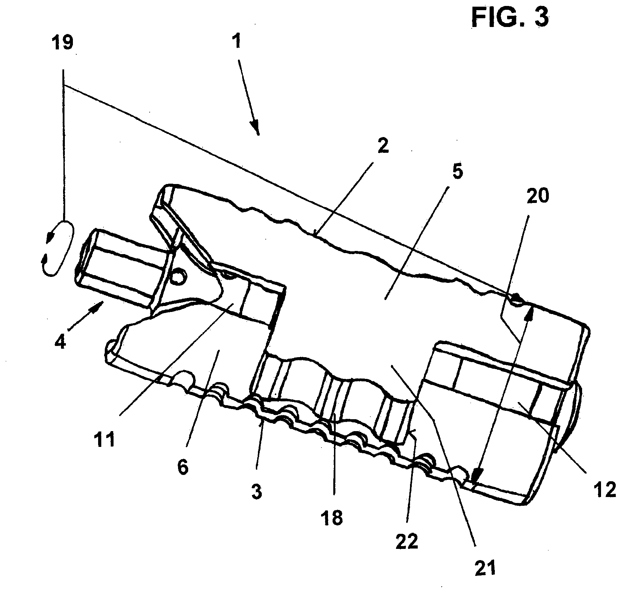

Wie besonders gut aus Fig. 3 ersichtlich, ermöglich die vorgenannte Konstruktion Folgendes: durch Rotation 19 des Führungselements 4 wird das das zweite Keilelement 12 ist in Richtung des ersten Keilelemens 11 verschoben und dadurch die beiden Spreizelemente 5 und 6 auseinander gedrückt. Die Breite 20 des Zwischenwirbel-Implantats 1 vergrössert sich und deren Stützflächen 2 und 3 legen sich an benachbarte Wirbelkörper der menschlichen Wirbelsäule und halten diese Wirbelkörper wirksam auseinander.

Aus Fig. 3 wird ausserdem ein weiteres technisches Merkmal dieser Konstruktion deutlich. Neben den Führungen 18 gibt es eine weitere Verbindung zwischen den beiden Spreizelementen 5 und 6. Diese ist als Steckverbindung ausgebildet und besteht in dieser Ausführung aus einer Auskragung 21 ersten Spreizelement 5, die verschiebbar in eine entsprechende Ausnehmung 22 im zweiten Spreizelement 6 greift. Möglich sind selbstverständlich auch mehr als eine Auskragung 21 und mehr als eine Ausnehmung 22, die auch wechselweise an den beiden Spreizelementen 5 und 6 angeordnet sein können. Obwohl die beiden Spreizelemente 5 und 6 des Zwischenwirbel-Implantats 1 hier nicht durch einen festen Steg oder dergleichen miteinander verbunden sind, werden sie durch die Steckverbindung mittels der Auskragung 21 und Ausnehmung 22 sicher in der geschlossenen Grundstellung nach Fig. 1 zusammen gehalten. Dadurch kann das Zwischenwirbel-Implantat 1 problemlos gehandhabt und operativ in einen Zwischenwirbelraum eingeführt werden.

Selbst in der Stützstellung nach Fig. 3 bleiben die beiden Stützflächen 2 und 3 optimal ausgerichtet. Sie liegen annähernd parallel zueinander und stützen die Wirbelsäule auf bestmögliche Weise, ohne diese in eine unerwünschte Fehlstellung zu drücken. Weitere Vorteile dieser Konstruktion: sowohl die Breite 20 als auch die Klemmspannung des Zwischenwirbel-Implantats 1 kann stufenlos eingestellt werden, notfalls auch nachträglich. Zudem ist das Zwischenwirbel-Implantat 1 jederzeit von der Stützstellung nach Fig. 3 in die Grundstellung nach Fig. 1 rückstell- und wieder extrahierbar.

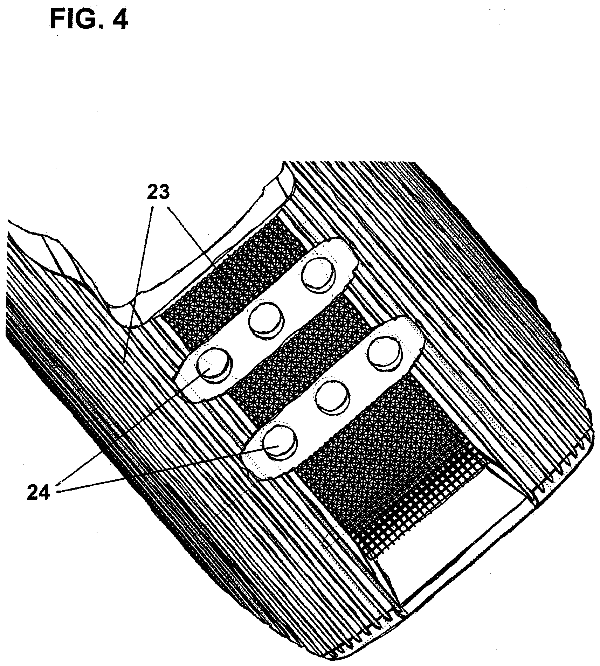

Wie in Fig. 4 dargestellt, kann das Zwischenwirbel-Implantat 1 an dessen Oberfläche mindestens teilweise eine raue und/oder poröse Struktur aufweisen. Es kann sich beispielsweise um Erhebungen 23 in Form von Rippen oder auch um spitze Erhebungen 23 handeln. Letztere können zum Beispiel pyramidenförmig sein. Eine weitere Möglichkeit liegt darin, mindestens einen Teil der Oberfläche des Zwischenwirbel-Implantats 1 mit Durchbrechungen 24 zu versehen, zum Beispiel Bohrungen. Diese können derart an einem oder an mehreren stegartigen Flanschen angeordnet sein, dass sich hinter oder unter den Durchbrechungen 24 ein Freiraum befindet. Diese Massnahmen dienen dazu, das Einwachsen des erfindungsgemässen Zwischenwirbel-Implantats 1 und dessen sicheren Halt im Zwischenwirbelraum zu begünstigen.

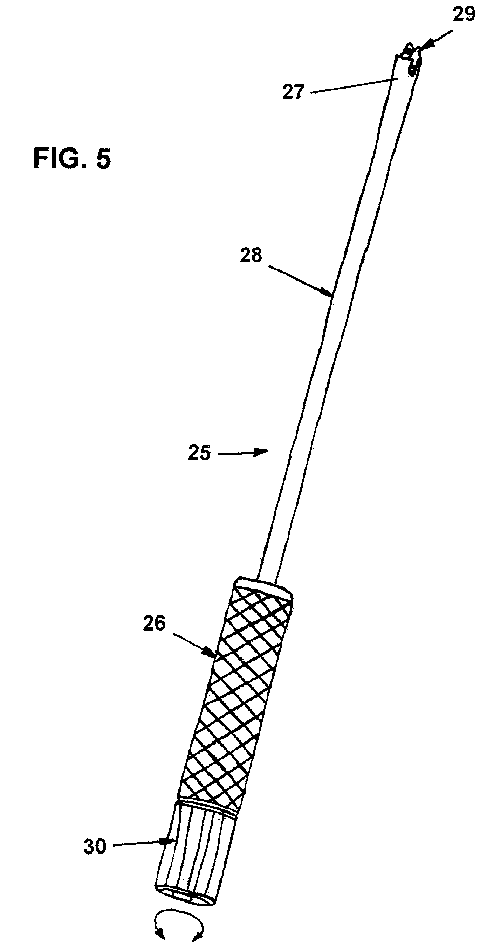

Unabhängig von der Ausführungsvariante kann das jeweilige Zwischenwirbel-Implantat 1 mittels eines Einbringinstruments 25 nach den Fig. 5 bis 8 in einen Zwischenwirbelraum eingeführt werden. Es sind mehrere unterschiedliche Ausführungen dieses Einbringinstruments 25 dargestellt.

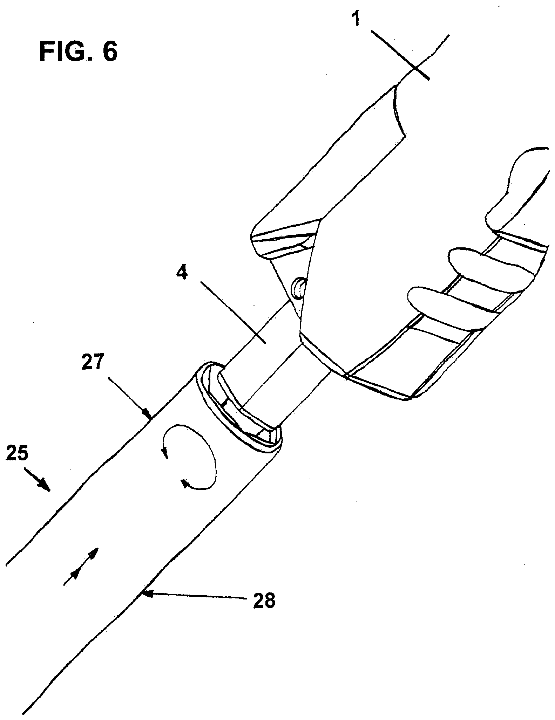

Das Einbringinstrument 25 weist einen Griff 26 auf und ist am distalen Ende 27 des Schaftes 28 zumindest dazu ausgelegt, mit dem Führungselement 4 des Zwischenwirbel-Implantats 1 zusammenzuwirken und dieses zu rotieren. Im bisher vorgestellten Fall, dass das Führungselement 4 als Bolzen mit dem Profil eines Mehrkants 16 ausgebildet ist, wird am distalen Ende 27 des Einbringinstruments 25 ein Innenmehrkant vorzusehen sein. Das entsprechende Detail ist aus Fig. 6 ersichtlich. Wie bereits erwähnt, bleibt jedoch auch die umgekehrte Anordnung dieser Vater/Mutter-Konstellation möglich.

Die Rotation des distalen Endes 27, und in der Folge des Führungselements 4 des Zwischenwirbel-Implantats 1, kann durch Rotation des Einbringinstruments 25 um seine eigene Längsachse mittels des Griffs 26 erfolgen.

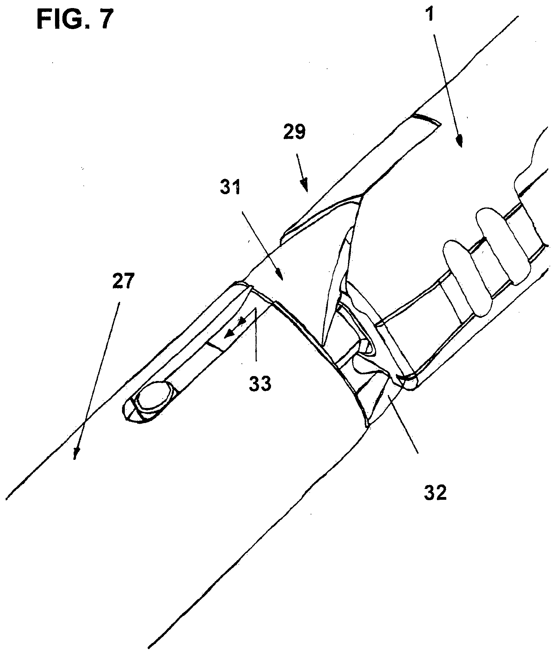

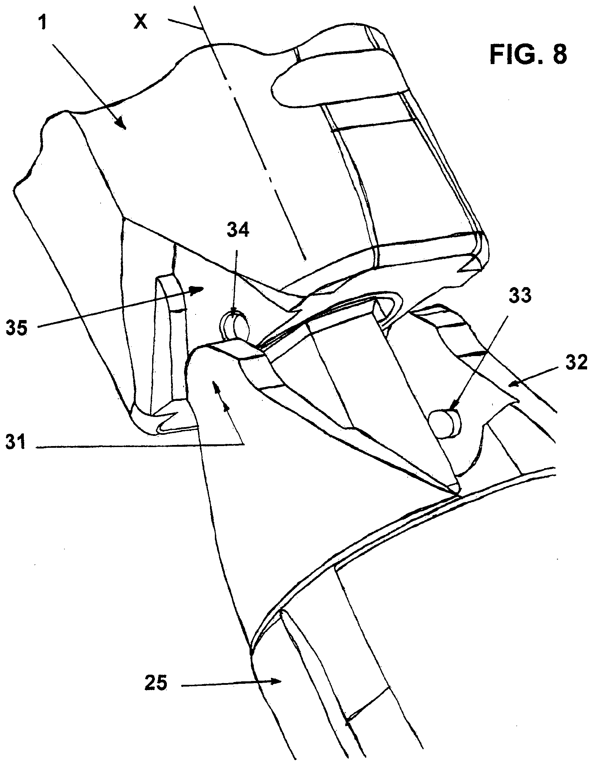

Gemäss den Fig. 7 und 8 kann in einer weiter gehenden Ausführung am distalen Ende 27 des Einbringinstruments 25 aber auch mindestens ein Greifelement 29 vorhanden sein. Dieses Greifelement 29 steht hier in Wirkverbindung mit einem am Ende des Griffes 26 angeordneten Handrad 30. Hierzu kann der Schaft 28 hohl ausgebildet sein, wobei das Handrad 30 mittels einer durch den in diesem Fall röhrenförmigen Schaft 28 geführte Antriebsstange mit dem Greifelement 29 verbunden ist.

Das Detail nach Fig. 7 zeigt wie am distalen Ende 27 des Einbringinstruments 25 ein Greifelement 29 in Form einer Spannzange mit zwei Zangenbacken 31 und 32 angeordnet sein kann. Denkbar ist aber auch ein Greifelement 29 mit mehr als zwei Zangenbacken. Diese Zangenbacken 31 und 32 sind hier mittels einer Zapfen-Rillen-Verbindung 33 am distalen Ende 27 geführt. Dabei sind die eigentlichen Zangenbacken 31 und 32 gegenüber ihrem hinteren, den Zapfen aufweisen Teil schwenkbar. Die Rille ist Teil des Schaftes 28.

Durch Zurückziehen in die in Fig. 7 sichtbare Schliessstellung werden die Zangenbacken 31 und 32 geschlossen und in dieser Schliessstellung gehalten. Diese Betätigung erfolgt wie gesagt über das Handrad 30. Die Fig. 7 veranschaulicht nicht zuletzt wie kompakt und fluchtend sich das Zwischenwirbel-Implantat 1 dem Einbringinstrument 25 anschliesst, was dessen operatives Einführen wesentlich erleichtert.

Die Momentaufnahme nach Fig. 8 stellt den Augenblick des Ankoppelns des Zwischenwirbel-Implantats 1 an das Einbringinstrument 25 oder umgekehrt dessen Auskoppeln dar. Hier ebenfalls gut zu sehen ist, dass die Zangenbacken 31 und 32 je einen Haltezapfen 33 aufweisen können, der je in ein entsprechendes Halteloch 34 am Zwischenwirbel-Implantat 1 eingreifen kann uns so dem sicheren Festhalten desselben dient. Das Zwischenwirbel-Implantat 1 weist im betreffenden Bereich vorzugsweise je eine Aussparung 35 auf, die annähernd den Umrissen der Zangenbacken 31 und 32 entspricht. Die Kontaktfläche dieser Aussparung 35 kann entsprechend dieser Darstellung eine schräge Gleitfläche aufweisen. Die dem Einbringinstrument 25 zuzuwendenden Enden der Gleitfläche der Aussparung 35 sind näher bei der Achse X des Zwischenwirbel-Implantats 1 als die dem Einbringinstrument 25 abzuwendenden Enden. Das heisst, zum Einbringinstrument 25 hin ist der Querschnitt dieses Bereichs des Zwischenwirbel-Implantats 1 kleiner und verbreitert sich zunehmend. Dadurch kann sowohl das Öffnen als auch das Positionieren der Zangenbacken 31 und 32 in der jeweiligen Aussparung 35 begünstigt werden.

Es liegt im Rahmen der Erfindung nach Patentanspruch 1 die einzelnen Bauteile des Zwischenwirbel-Implantats 1 auch anders als im Ausführungsbeispiel gezeichnet auszubilden. So können die Keilelemente 11 und 12 anders geformt sein, solange im Zusammenspiel mit den Spreizelementen 5 und 6 ein Auseinanderdrücken erreicht wird. Dasselbe gilt für das Einbringinstrument in Bezug auf den Patentanspruch 5.

## Patentansprüche

1. Zwischenwirbel-Implantat mit an benachbarten Wirbelkörpern anlegbaren Stützflächen (2, 3), die bezüglich ihrer Lage zueinander verstellbar sind, mit mindestens zwei je mindestens eine Keilfläche (9, 10) oder einen Anschlag aufweisende Keilelemente (11, 12), wovon mindestens eines entlang einer annähernd parallel zu den Stützflächen (2, 3) liegenden Achse (X) eines Verstellelements (13) verschiebbar ist, wobei mit den Stützflächen (2, 3) verbundene Spreizelemente (5, 6) den Keilelementen (11, 12) zugewandte Anschläge (7, 8) oder Keilflächen aufweisen, die als spitzwinklig zur Achse (X) des Verstellelements (13) liegenden Schrägen ausgebildet sind, wodurch die beiden mittels einer Steckverbindung ineinander greifenden Spreizelemente (5, 6) durch Veränderung der relativen Lage der Keilelemente (11, 12) parallel auseinander drückbar und eine Breite (20) des Zwischenwirbel-Implantats (1) veränderbar ist, wobei die Spreizelemente (5, 6) mittels Führungen (18) ineinander greifen, die an jeweils gleitend einander zugeordneten Flächen dieser beiden Spreizelemente (5, 6) ausgeformt und rechtwinklig zur Achse (X) des Verstellelementes (13) ausgerichtet sind, wobei mindestens ein Teil der Oberfläche mit Durchbrechungen versehen ist, **dadurch gekennzeichnet, dass** die Durchbrechungen (24) an mindestens einem stegartigen Flansch angeordnet sind, wobei sich hinter oder unter ihnen ein Freiraum befindet.

2. Zwischenwirbel-Implantat nach Anspruch 1, **dadurch gekennzeichnet, dass** die Spreizelemente (5, 6) mittels einer Steckverbindung ineinander greifen und dadurch zusammen gehalten werden, wobei ein erstes Spreizelement mindestens eine Auskragung (21) aufweist, die verschiebbar in mindestens eine entsprechende Ausnehmung (22) im zweiten Spreizelement (6) greift.

3. Zwischenwirbel-Implantat nach Anspruch 1 oder 2, **dadurch gekennzeichnet, dass** das Verstellelement (13) an mindestens einem Ende ein Führungselement (4) aufweist, an das ein Einbringinstrument (25) zur rotierenden Betätigung des Verstellelements (13) und zum Halten des Zwischenwirbel-Implantats (1) ansetzbar ist, wobei sowohl die Breite (20) als auch die Klemmspannung des Zwischenwirbel-Implantats stufenlos einstellbar ist und es von einer Stützstellung in eine Grundstellung rückstell- und wieder extrahierbar ist.

4. Zwischenwirbel-Implantat nach Anspruch 3, **dadurch gekennzeichnet, dass** das Führungselement (4) einen Mehrkant (17) aufweist, zum Beispiel einen Aussen- oder Innensechskant.

5. Zwischenwirbel-Implantat nach einem der Ansprüche 1 - 4, **gekennzeichnet durch** mindestens zwei Haltelöcher (34) zum Festhalten des Zwischenwirbel-Implantats (1) mittels eines Einbringinstrumentes (25).

## Claims

1. An interbody implant with support surfaces (2, 3) that can be applied to adjacent vertebral bodies and can be repositioned relative to each other, with at least two wedge elements (11, 12), each having at least one wedge surface (9, 10) or a limit stop, of which at least one wedge element is displaceable along an axis (X) of an adjustment element (13) which extends approximately parallel to the support surfaces (2, 3), wherein spreading elements (5, 6) connected to the support surfaces (2, 3) have limit stops (7, 8) or wedge surfaces which face towards the wedge elements (11, 12) and are constructed to slant at an acute angle to the axis (X) of the adjustment element (13), so that the two spreading elements (5, 6), which mesh with each other by means of a push-in connection, can be pressed apart in parallel by changing the relative position of the wedge elements (11, 12), and a width (20) of the interbody implant (1) can be altered, wherein the spreading elements (5, 6) mesh with each other with the aid of guides (18) which are conformed on surfaces of these two spreading elements (5, 6), each of which is assigned in sliding manner to the other and aligned at right angles to the axis (X) of the adjustment element (13), wherein at least a part of the surface is furnished with perforations (24), **characterized in that** the perforations (24) are provided on at least one fin-like flange, wherein a free space is located behind or below them.

2. The interbody implant according to Claim 1, **characterized in that** the spreading elements (5, 6) mesh with each other by means of a push-in connection and are held together thereby, wherein a first spreading element has at least one projection (21) which can be pushed into at least one corresponding recess (22) to engage in the second spreading element (6).

3. The interbody implant according to Claim 1 or 2, **characterized in that** at least one end of the adjustment element (13) has a guide element (4) to which an insertion tool (25) can be attached for rotating actuation of the adjustment element (13) and for holding the interbody implant (1), wherein both the width (20) and the clamping tension of the interbody implant is steplessly adjustable, and it can be retracted from a support position to a neutral position and extended again.

4. The interbody implant according to Claim 3, **characterized in that** the guide element (4) has a multi-edge form (17), for example a hexagonal external or internal contour.

5. The interbody implant according to any one of Claims 1 to 4, **characterized by** at least two retaining holes (34) for holding the interbody implant (1) in place by means of an insertion tool (25).

## Revendications

1. Implant intervertébral comportant des surfaces d'appui (2, 3) pouvant être posées au niveau de corps vertébraux voisins et qui sont réglables les unes par rapport aux autres pour ce qui est de leur position, comportant au moins deux éléments cunéiformes (11, 12) présentant chacun au moins une surface cunéiforme (9, 10) ou une butée, dont au moins une déplaçable le long d'un axe (X) d'un élément de réglage (13) situé approximativement parallèlement aux surfaces d'appui (2, 3), des éléments d'écartement (5, 6) raccordés aux surfaces d'appui (2, 3) présentant des butées (7, 8) tournées vers les éléments cunéiformes (11, 12) ou des surfaces cunéiformes qui sont réalisées sous forme d'obliques situées en angle aigu par rapport à l'axe (X) de l'élément de réglage (13), ce qui a pour effet que les deux éléments d'écartement (5, 6) s'engrenant dans l'un dans l'autre au moyen d'une connexion enfichable peuvent être poussés parallèlement loin l'un de l'autre par modification de la position relative des éléments cunéiformes (11, 12) et qu'une largeur (20) de l'implant intervertébral (1) est modifiable, les éléments d'écartement (5, 6) s'engrenant l'un dans l'autre au moyen de guides (18) qui sont formés au niveau de surfaces respectivement associées en coulissement l'une à l'autre de ces deux éléments d'écartement (5, 6) et sont orientés orthogonalement par rapport à l'axe (X) de l'élément de réglage (13), au moins une partie de la surface étant pourvue de percées (24), **caractérisé en ce que** les percées (24) sont disposées au niveau d'au moins une bride en forme de traverse, un espace libre se trouvant derrière ou en-dessous d'elles.

2. Implant intervertébral selon la revendication 1, **caractérisé en ce que** les éléments d'écartement (5, 6) s'engrènent les uns dans les autres au moyen d'une connexion enfichable et sont ainsi maintenus ensemble, un premier élément d'écartement présentant au moins une collerette (21) qui s'engrène de manière déplaçable dans au moins un évidement correspondant (22) du second élément d'écartement (6).

3. Implant intervertébral selon la revendication 1 ou 2, **caractérisé en ce que** l'élément de réglage (13) présente à au moins une extrémité un élément de guidage (4) au niveau duquel un instrument d'introduction (25) peut être posé pour l'actionnement en rotation de l'élément de réglage (13) et pour la retenue de l'implant intervertébral (1), aussi bien la largeur (20) que la tension de serrage de l'implant intervertébral étant réglables progressivement et celui-ci pouvant être réinitialisé et de nouveau extrait depuis une position d'appui jusqu'à une position de base.

4. Implant intervertébral selon la revendication 3, **caractérisé en ce que** l'élément de guidage (4) présente un polygone (17), en particulier un hexagone extérieur ou intérieur.

5. Implant intervertébral selon une des revendications 1 à 4, **caractérisé par** au moins deux trous de retenue (34) pour maintenir l'implant intervertébral (1) au moyen d'un instrument d'introduction (25).
